# EUROPÄISCHE PATENTSCHRIFT

(11) **EP 0 617 120 B1**
(45) Veröffentlichungstag und Bekanntmachung des Hinweises auf die Patenterteilung: **31.05.2000**
(21) Anmeldenummer: 94810109.2
(22) Anmeldetag: 22.02.1994
(51) Int. Cl.: C12M 1/107, C12M 1/02

(54) **Fermentationseinrichtung**
Fermentation device
Dispositif de fermentation

(30) Priorität: 25.02.1993 CH 57493
(43) Veröffentlichungstag der Anmeldung: 28.09.1994
(73) Patentinhaber: LINDE BRV Biowaste Technologies AG, 6300 Zug (CH); Schmutz, Urs, 4466 Ormalingen (CH)
(72) Erfinder: Rindelaub, Frank Alex Erich, 2014 Bôle (CH); Schmutz, Urs, 4466 Ormaligen (CH)
(74) Vertreter: Braun, André

(56) Entgegenhaltungen:
- DE-A- 3 239 304
- FR-A- 2 481 873
- NL-A- 8 700 588

## Beschreibung

Die Erfindung betrifft eine Fermentationseinrichtung zum biologischen Abbau von organscher Materie und zur Gewinnung des beim Abbau entstehenden Biogases mit einem geschlossenen Behälter mit einer Einbringöffnung für Frischgut und Umwälz- oder Impfgut und Austragsöffnungen für die Abbauprodukte und mit einer Rühreinrichtung zum Durchmischen des Fermentationsgutes.

Der Begriff Fermentation wird für den Zweck dieser Beschreibung in einem allgemeinen Sinne verwendet und umfasst aerobe und anaerobe, sowie gesteuerte und nicht gesteuerte Biotransformationsprozesse, wie Gärung etc.

Mit dem Begriff Ferinentationsgut wird in der vorliegenden Beschreibung die im Fermentationsprozess befindliche Materie bezeichnet.

Mit dem Begriff Frischgut wird die abzubauende organische Materie bezeichnet, der Begriff Impfgut steht für organische Materie, die dem Fermentationsprozess entnommen wird und die für die Fermentation notwendigen Mikroorganismen und Enzyme enthält, und die bei diskontinuierlichen Prozessen dem Frischgut zugefügt wird, um die Fermentation einzuleiten.

Fermentationseinrichtungen der eingangs genannten Art sind bekannt, unter anderem unter der Bezeichnung Gärgasanlagen. Bei derartigen Anlagen werden die als Frischgut unter teilweiser Beimischung von Impfgut einem Fermenter zugeführten Stoffe vorgängig oder im Fermenter auf Prozesstemperatur erwärmt und während des Prozesses von einem Rührsystem durchmischt. Die entstehenden Gase können beispielsweise einer Energieversorgungsanlage zugeführt oder auch zur Deckung der eigenen Prozessenergie verwendet werden.

Aus FR-A-2481873 und DE-A-3239 304 sind Fernender für Flüssigkeiten bekannt. Bei diesen sind die Technischen Probleme der Durchmischung und Prozesssteurung weitgehend gelöst.

Die bekannten Gärgasanfagen haben den Nachteil, dass die umlaufenden Rührwerke technisch, d.h. vor allem mechanisch, sehr aufwendig sind und viel Energie konsumieren und dass trotzdem homogene Durchmischung und eine für den Prozess optimale Temperaturverteilung im Fermentationsgut nicht gewährleistet ist.

Der Erfindung liegt die Aufgabe zugrunde, diese Nachteile zu beheben, indem eine mechanisch einfachere und damit kostengünstiger herzustellende und zu betreibende Fermentationseinrichtung bereitgestellt wird, die zudem optimale Durchmischung und Wärmeverteilung gewährleistet.

Erfindungsgemäss wird diese Aufgabe dadurch gelöst, dass die Rühreinrichtung kreisförmig bewegte Rührflügel aufweist. Die Rührflügel können entweder mit einem sie simultan im Behälter bewegenden Antriebsmechanismus verbunden oder individuell, d.h. unabhängig voneinander angetrieben sein.

Die Rührflügel bestehen vorzugsweise aus mit Trägern und Querstreben an Wellen befestigten Schaufeln, die quer zur senkrechten Mittelebene des Behälters angeordnet sind.

Gemäss einer bevorzugten Ausführungsform der Erfindung ist an den Seitenwänden ein Durchlaufsystem für ein Heizmedium vorgesehen, das in mehrere Heizkreise aufgeteilt sein kann, um die Temperatur an verschiedenen Sektoren der Heizfläche zu variieren. Vorzugsweise ist das Durchlaufsystem aussen an den Behälterwänden angebracht.

Gemäss einer weiteren bevorzugten Ausführungsform der Erfindung sind am Behälterboden Scharren angebracht, mit denen auf dem Behälterboden abgelagerte Sinkstoffe schubweise zu einer Austragsvorrichtung befördert werden. Diese Scharren können entweder mit dem Antrieb der Rührflügel gekoppelt sein oder zwecks flexibler Anpassung der Förderleistung ebenfalls getrennte Antriebe besitzen.

Im Folgenden werden anhand der beiliegenden Zeichnungen bevorzugte Ausführungsbeispiele der Erfindung beschrieben. Es zeigen
- Fig. 1: eine Fermentationseinrichtung in Seitenansicht mit abgeschnittener Behälterseitenwand
- Fig. 2: eine Fermentationseinrichtung in Draufsicht mit abgeschnittener Behälterdecke
- Fig. 3: eine Fermentationseinrichtung in Frontansicht mit abgeschnittener Behälterfrontwand
- Fig. 4: Details der Rühreinrichtung
- Fig. 5: ein weiteres Detail der Rühreinrichtung
- Fig. 6: eine Anordnung von mehreren miteinander verbundenen Einrichtungen zur Vorkompostierung, Fermentation und Nachkompostierung

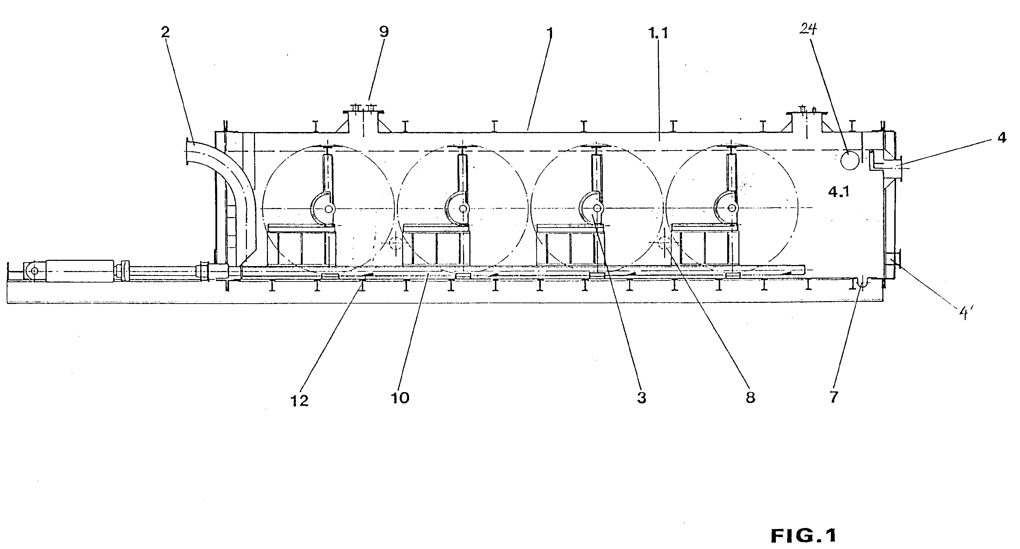

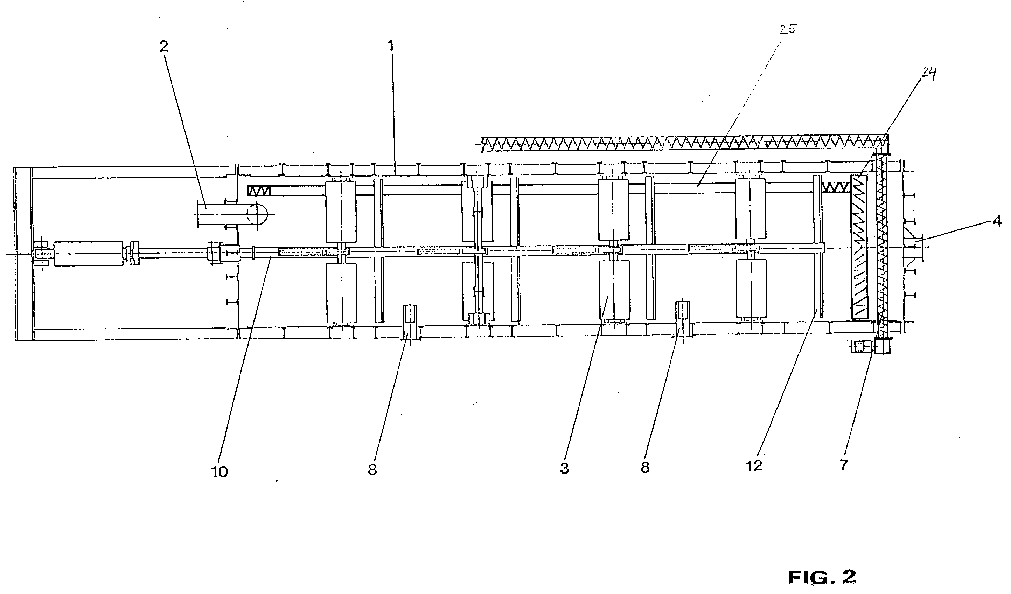

Die in Fig. 1 und 2 gezeigte Fermentationseinrichtung besteht aus einem rechteckigen geschlossenen Behälter 1 mit einem von ebenen Wänden begrenzten Innenraum 1.1 zur Aufnahme des Fermentationsgutes 4.1. Der Behälter besitzt eine Einbringöffnung 2 für Frischgut und Umwälz- oder Impfgut und Austragsöffnungen 4 und 7 für die Abbauprodukte. Der Behälter weist ferner in den Seitenwänden angeordnete Probenentnahmestutzen 8, sowie in seiner Decke angeordnete Gasentnahmestutzen 9 auf.

Im Behälter befindet sich eine Rühreinrichtung 3 zur Durchmischung des Fermentationsgutes und eine Kratzbodeneinrichtung 12 für den Austrag abgelagerter schwerer Sinkstoffe, wie Sand, Steine, Metallteile etc.

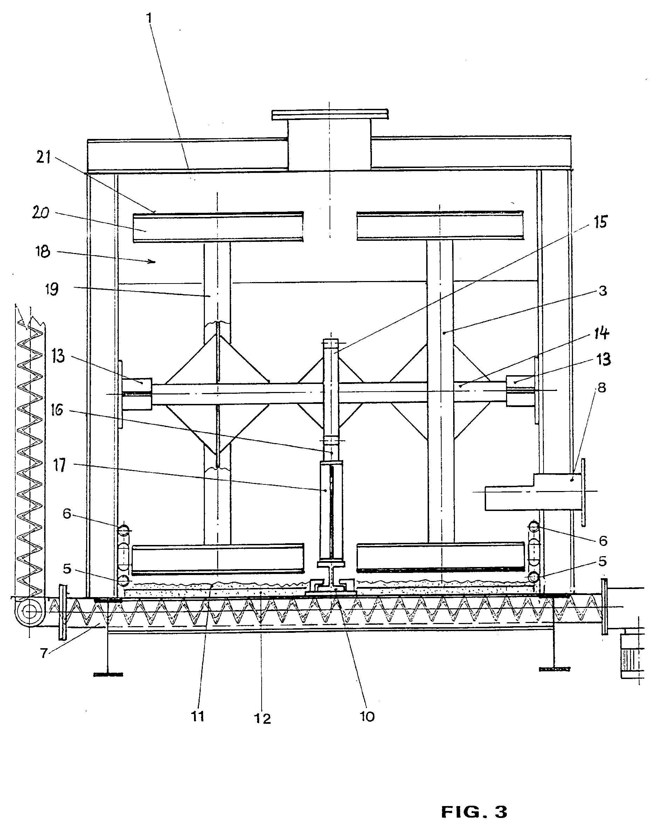

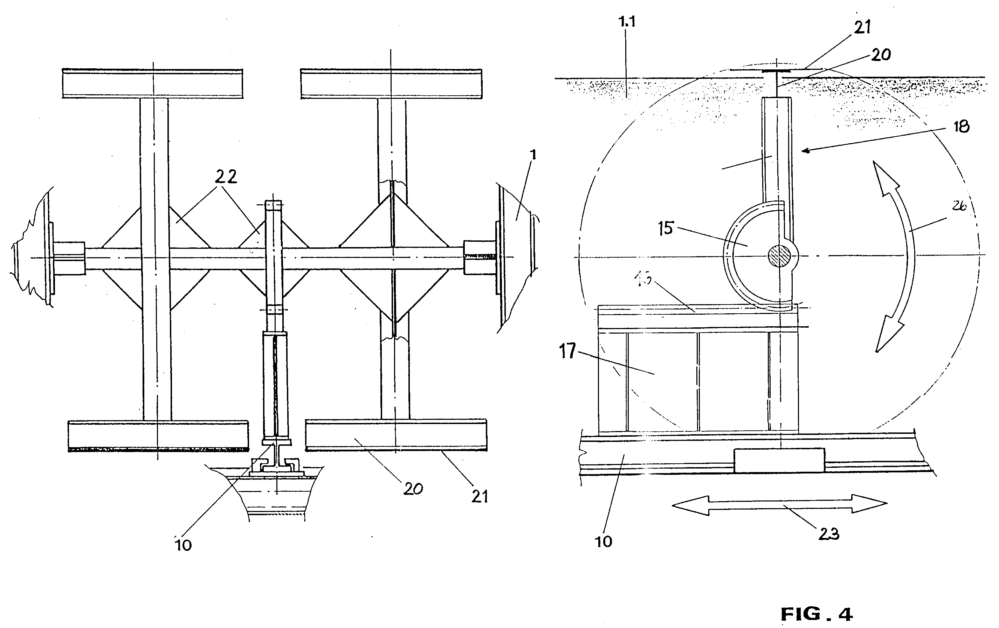

Die Durchmischung wird durch ein System von Rührflügeln bewirkt, die in den Fig. 3 und 4 detailliert gezeigt sind.

Wie am besten aus Fig. 3 ersichtlich, befinden sich Lager 13 paarweise auf etwa halber Höhe der Innenseiten der Seitenwände des Behälters 1. In einem Lagerpaar 13 ist eine Welle 14 gelagert. Insgesamt sind, gleichmässig über die Länge des Behälters 1 verteilt, vier auf diese Weise zwischen den Seitenwänden angeordnete Wellen 14 vorgesehen.

In ihrer Mitte ist auf den Wellen ein Antriebszahnrad 15 angebracht, das im vorliegenden Fall einen Sektor von etwa 180 Grad umfasst. Das Zahnrad greift in eine entsprechend gezahnte, linear angetriebene Zahnschiene 16 ein. Die Zahnschiene 16 ist über eine Stütze 17 mit einer Schubstange 10 verbunden, die sich über die ganze Länge des Behälters 1 erstreckt und von einer ausserhalb des Behälters angeordnete Antriebsvorrichtung hin- und herbewegt wird. Selbstverständlich können alternativ auch andere Antriebsarten, z.B. individuelle hydraulische Antriebe für jede Welle etc., zum Einsatz kommen.

Jeweils in der Mitte zwischen Antriebszahnrad und den Lagern sind Rührflügel 18 angebracht. Diese bestehen aus einem senkrecht zur Welle 14 an ihr befestigten Träger 19, an dessen beiden Enden sich je eine Querstrebe 20 und eine an dieser befestigte, zur Welle parallele Schaufel 21 befinden. Träger 19 und Strebe 20 bestehen im vorliegenden Ausführungsbeispiel aus H-Profilen, die Schaufel 21 besteht aus Blech. Die Verbindung zwischen Welle und Trägern, wie auch zwischen Welle und Zahnradsektor ist vorzugsweise mit Blechwinkeln 22 verstärkt.

Die Durchmischungsfunktion ist am besten aus Fig. 4 b ersichtlich. Durch lineare Verschiebung der Schubstange 10 und damit der Stütze 17 und der Zahnschiene 16 gemäss dem Pfeil 26 nach rechts wird der Antriebszahnradsektor 15 und damit die Welle 14 und die Rührflügel 18 gemäss dem Pfeil 24 im Gegenuhrzeigersinn gedreht. Bei dieser Drehung wird die oben befindliche Schaufel durch das Fermentationsgut nach unten gezogen und dabei in den oberen Schichten befindliches Material nach unten gebracht. Gleichzeitig wird die zunächst unten befindliche Schaufel und durch sie Material von unten nach oben gezogen. Diese Materialumwälzung bewirkt eine ausgezeichnete Durchmischung des Fermentationsgutes. Der Vorgang wird in zeitlichen Abständen nach Bedarf wiederholt, also beispielsweise immer dann, wenn die den Fermentationsprozess begleitende Gasentwicklung nachlässt oder aufhört.

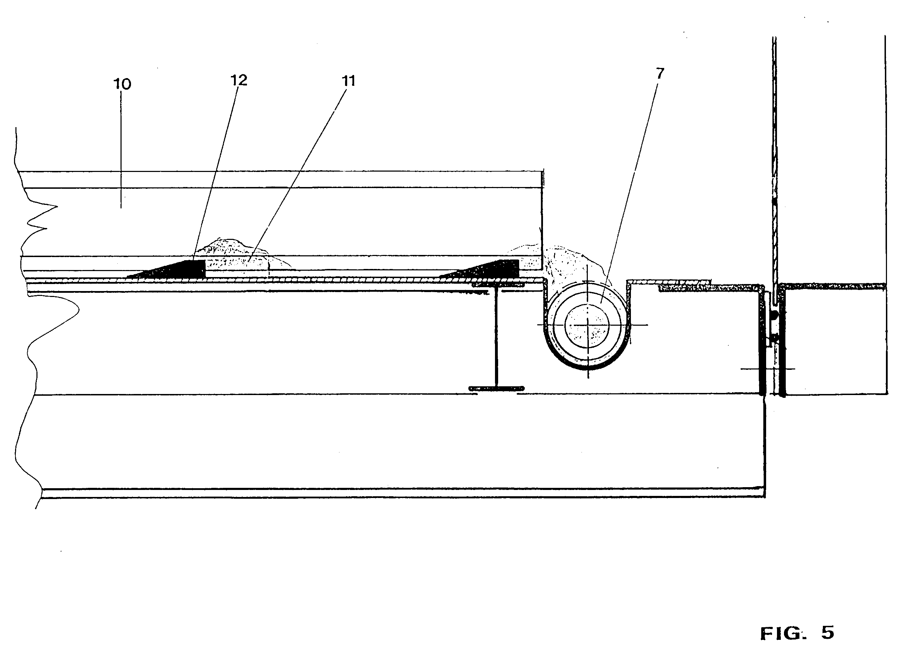

Der Austrag von abgelagerten schweren Sinkstoffen 11 wird durch Scharren 12 bewirkt, die am besten in Fig. 5 ersichtlich sind. Die Scharren 12 sind mit der Schubstange 10 verbunden und gleiten auf dem Boden des Behälters 1. Sie besitzen einen keilförmigen Querschnitt mit rampenförmiger Rückseite und steiler Front. Bei der Rückbewegung (in Fig. 5 nach links) gelangen die abgelagerten Sinkstoffe über die Rampenfläche hinweg auf die Vorderseite der Scharre. Bei der nächsten Vorwärtsbewegung erfolgt dann mit der steilen Frontseite die Förderung in Richtung zur Austragschnecke 7.

Alternativ können die Scharren 12 die Form von Klappen haben, die sich bei der linearen Rückwärtsbewegung der Rühreinrichtung (in Fig. 5 nach links) anheben und so über das abgelagerte Material hinweggleiten, während sie sich bei der Vorwärtsverschiebung der Rühreinrichtung (in Fig. 5 nach rechts) absenken und die Ablagerungen 11 in die Nähe der Austragsschnecke 7 schieben.

Die Scharren 12 können vorzugsweise unabhängig vom Antrieb der Rührflügel betätigt werden.

Wie am besten in Fig. 3 ersichtlich ist entlang den Seitenwänden im unteren Bereich ein Durchlaufsystem für ein Heizmedium mit einer Vorlaufleitung 5 und einer Rücklaufleitung 6 angeordnet. Vom Heizdurchlaufsystem erfolgt die Wärmeübertragung direkt an das Fermentationsgut. Infolge der Auf- und Abbewegung der Rühreinrichtung ergibt sich eine dynamische Wärmeübertragung. Dadurch wird das Fermentationsgut auf die erforderliche Prozesstemperatur erwärmt und werden Abstrahlungsverluste über den Behältermantel kompensiert.

Das Durchlaufsystem für das Heizmedium kann in mehrere Heizkreise aufgeteilt sein, die mit unterschiedlichen Heizleistungen beaufschlagt werden können, um die Temperatur an verschiedenen Sektoren der Heizfläche zu variieren. Auf diese Weise ergibt sich in der Steuerung des Temperaturverlaufes grössere Flexibilität zur optimalen Anpassung an die Anforderungen des Prozesses.

Alternativ zur innenliegenden Heizung sind die Heizmedium-Leitungen aussen auf den Behälterwänden angeordnet. Dies hat den Vorteil, dass die Innenwände glatt sind und weniger Materialablagerung möglich ist.

Da zu Beginn des Prozesses das frisch eingebrachte Fermentationsgut aufgeheizt werden muss und ausserdem zum Schutz der Bakterien nur mit geringen Temperaturdifferenzen geheizt werden sollte, ist im Bereich der ersten Rührflügel eine besonders grosse Heizfläche zweckmässig, die auch die Stirnwand einbezieht. Im weiteren Verlauf der Fermentation brauchen nur noch die Wärmeverluste ersetzt werden, so dass die von aussen zugeführte Wärme von Stufe zu Stufe verringert werden kann. Die aussenliegenden Heizungselemente können daher immer kleiner werden. Die Heizungselemente sind vorzugsweise in Form von doppelwandigen Durchlaufsystemen ausgeführt.

Durch die Bewegung der Rührflügel 18 wird das Fermentationsgut durchmischt und durch die auftretenden Scherkräfte zerteilt. Dadurch, dass die Rühreinrichtung sich weitgehend über den gesamten vom Fermentationsgut ausgefüllten Innenraum 1.1 erstreckt, findet optimale Durchmischung des gesamten Fermentationsgutes statt, was zusammen mit der erwähnten verbesserten Heizung zu hoher Prozessstabilität und wirksamer Entgasung des Fermentationsgutes führt.

Für die Wirksamkeit der Rühreinrichtung 3 ist ein rechteckiger oder vieleckiger Querschnitt des Behälters mit ebenen Wänden optimal.

In dem Raum zwischen den letzten Rührflügeln und den Austragsöffnungen 4, 4' befindet sich das Fermentationsgut in relativer Ruhe, so dass sich an der Oberfläche ein sehr fester, schwimmender Kuchen bilden könnte, der nicht mehr ohne weiteres durch die Öffnung 4 ausgetragen werden könnte. Um dies zu vermeiden ist in diesem Bereich eine waagerechte Förderspirale 24 quer zur Längsachse der Anlage angeordnet. Diese Förderspirale hat zwei gegensinnig gewundene Hälften derart, dass bei einheitlicher Drehrichtung Material von den beiden seitlichen Bereichen zur Mitte hin gefördert wird.

Eine weitere Rohrförderspirale 25 erstreckt sich in Längsrichtung durch die Anlage und dient der Förderung von Material aus dem Endbereich zurück zum Anfangsbereich der Anlage. Diese Rückmischung von bereits fermentiertem Material zum Frischgut dient der Beeinflussung physikalischer und biotechnologischer Prozessparameter. Die Rückmischungsspirale kann entweder im Boden oder im oberen Bereich des Fermentationsgutes, seitlich oder in der Mitte angeordnet sein.

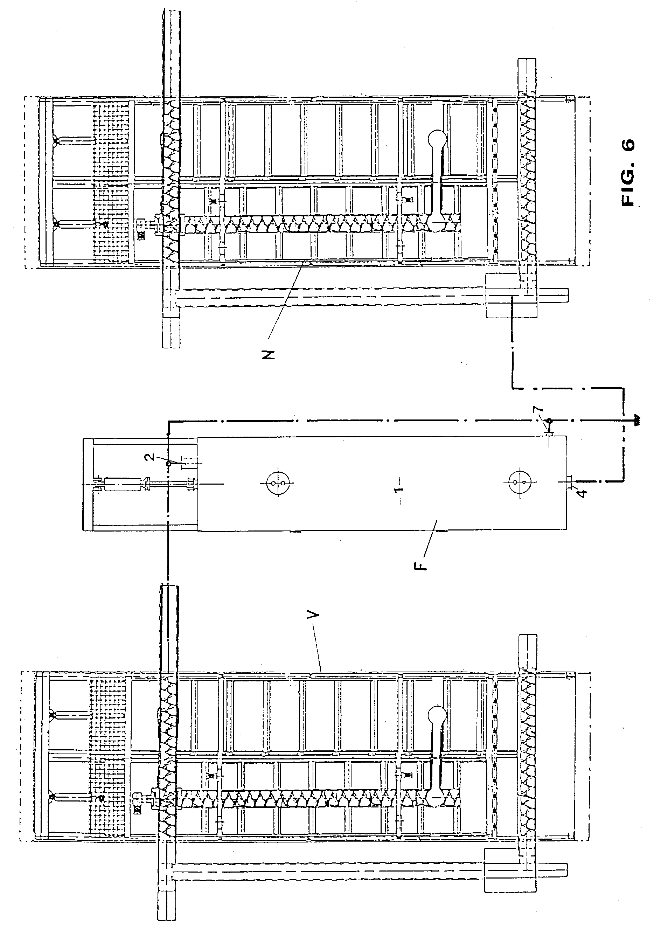

Fig. 6 zeigt die vorteilhafte Anordnung der vorliegenden Fermentationseinrichtung F im Verbund mit einer vorgeschalteten Kompostiereinrichtung V und einer nachgeschalteten Kompostiereinrichtung N. Je nach Grösse der Kompostiereinrichtungen kann es sinnvoll sein, mehrere Fermentationseinrichtungen nach der vorliegenden Erfindung parallel zu schalten. Geeignete Kompostiereinrichtungen sind beispielsweise Gegenstand von EP-A-0 592 368.

## Patentansprüche

1. Fermentationseinrichtung zum biologischen Abbau von organischer Materie und zur Gewinnung des beim Abbau entstehenden Biogases mit einem geschlossenen Behälter mit einer Einbringöffnung für Frischgut und Umwälz- oder Impfgut und Austragsöffnungen für die Abbauprodukte und mit einer Rühreinrichtung zum Durchmischen des Fermentationsgutes, dadurch gekennzeichnet, dass die Rühreinrichtung (3) kreisförmig bewegte Rührflügel (18) aufweist die individuell angetrieben sind.

2. Fermentationseinrichtung nach Anspruch 1, dadurch gekennzeichnet, dass die Rührflügel (18) aus Trägern (19), Querstreben (20) und Schaufeln (21) bestehen, die an Wellen (14) befestigt sind, die quer zur senkrechten Mittelebene des Behälters angeordnet sind.

3. Fermentationseinrichtung nach einem der Ansprüche 1 - 2, dadurch gekennzeichnet, dass an den Seitenwänden ein Durchlaufsystem (5, 6) für ein Heizmedium vorgesehen ist.

4. Fermentationseinrichtung nach Anspruch 3, dadurch gekennzeichnet, dass das Durchlaufsystem für das Heizmedium in mehrere Heizkreise aufgeteilt ist, um die Temperatur an verschiedenen Sektoren der Heizfläche zu variieren.

5. Fermentationseinrichtung nach einem der vorangehenden Ansprüche, dadurch gekennzeichnet, dass auf dem Boden des Behälters Scharren (12) angeordnet sind, mit denen auf dem Behälterboden abgelagerte Sinkstoffe (11) schubweise zu einer Austragsvorrichtung (7) befördert werden.

6. Fermentationseinrichtung nach einem der vorangehenden Ansprüche gekennzeichnet durch ein in Längsrichtung im Gehäuse angeordnetes Transportmittel zur Förderung von Fermentationsgut aus dem Endbereich zum Eintragsbereich.

## Claims

1. Fermentation device for the biological decomposition of organic material and for obtaining the biogas resulting from the decomposition with a closed container with a feed opening for fresh products and circulating products or inoculants and a discharge opening for the decomposition products and with a stirring device for mixing the fermentation product, characterized in that the stirring device (3) has agitator blades (18) driven circularly which are operated individually.

2. Fermentation device according to claim 1, characterized in that the agitator blade (18) consists of supports (19), cross struts (20) and blades (21), which are attached to the spindles (14), which are arranged diagonally to the vertical centre plane of the container.

3. Fermentation device according to one of claims 1 - 2, characterized in that a flow system (5, 6) for a heating means is provided on the side walls

4. Fermentation device according to claim 3, characterized in that the flow system for the heating means Is divided into several heating circuits, in order to vary the temperature in different sectors of the heating surface.

5. Fermentation device according to one of the previous claims, Characterized in that scrapers (12) are arranged on the bottom of the container, with which sediments (11) deposited on the bottom of the container are conveyed in batches to the discharge device (7).

6. Fermentation device according to one of the previous claims, characterized by a means of transport arranged lengthwise in the housing for conveying fermentation products from the end area into the feed area.

## Revendications

1. Dispositif de fermentation pour la décomposition biologique de matières organiques et la récupération du biogaz issu de cette décomposition présentant un réservoir fermé ayant un orifice de chargement pour les produits frais et les produits de circulation ou d'inoculation et des orifices de déchargement pour les produits de décomposition et présentant un dispositif agitateur destiné à réaliser un mélange complet du produit de fermentation, caractérisé en ce que le dispositif agitateur (3) présente des palettes d'agitation (18) ayant un mouvement circulaire et entraïnées séparément.

2. Dispositif de fermentation selon la revendication 1, caractérisé en ce que les palettes d'agitation (18) sont constituées de supports (19), de barres transversales (20) et de pales (21) fixés à des arbres (14) qui sont disposés de manière transversale par rapport au plan médian vertical du réservoir.

3. Dispositif de fermentation selon la revendication 1 ou la revendication 2, caractérisé en ce qu'un système de circulation (5, 6) pour un fluide chaud est prévu sur les parois latérales.

4. Dispositif de fermentation selon la revend;cation 3, caractérisé en ce que le système de circulation pour le fluide chaud est divisé en plusiers cycles calorifiques, afin de faire varier la température au niveau des différents secteurs de la surface de chauffe.

5. Dispositif de fermentation selon l'une quelconque des revendications précédentes, caractérisé en ce que des racloirs (12) sont disposés au fond du réservoir et permettent d'acheminer par petits paquets les sédiments (11) déposés au fond du réservoir vers un dispostif de déchargement (7).

6. Dispositif de fermentation selon l'une quelconque des revendications précédentes, caractérisé par un moyen de transport disposé dans le bâti dans la direction longitudinale pour acheminer les produits de fermentation de la zone terminale à la zone d'admission.
